# FASCICULE DE BREVET EUROPEEN

(11) **EP 0 721 110 B1**
(45) Date de publication et mention de la délivrance du brevet: **28.05.2003**
(21) Numéro de dépôt: 96440002.2
(22) Date de dépôt: 08.01.1996
(51) Int. Cl.: G01R 33/567, A61B 5/113

(54) **Dispositif capteur délivrant notamment un signal représentatif de la respiration d'un patient**
Sensor besonders zur Abgabe eines Signales zur Darstellung der Atmung eines Patienten
Sensor for especially delivering a signal representative of the respiration of a patient

(30) Priorité: 09.01.1995 FR 9500335
(43) Date de publication de la demande: 10.07.1996
(73) Titulaire: Schiller Medical, 67160 Wissembourg (FR)
(72) Inventeur: Cansell, Albert, F-67160 Wissembourg (FR); Kraemer, Michel, F-67360 Durrenbach (FR); Brevard, Christian, F-67160 Wissembourg (FR); Felblinger, Jacques, CH-3013 Bern (CH); Boesch, Chris, CH-3600 Thun (CH)
(74) Mandataire: Metz, Paul

(56) Documents cités:
- EP-A- 0 498 996
- WO-A-87/00922
- WO-A-94/23648
- US-A- 4 751 462

## Description

La présente invention concerne le domaine du prélèvement et de la mesure de signaux biologiques et de la surveillance de patients, notamment de patients sous examen RMN (Résonance Magnétique Nucléaire) par exemple dans un Imageur à Résonance Magnétique (IRM), et a pour objet un dispositif capteur délivrant notamment un signal représentatif de la respiration d'un patient.

Actuellement, les mouvements respiratoires et/ou le flux respiratoire d'un patient sont principalement relevés au moyen, soit de dispositifs capteurs électroniques spécifiques, mesurant le flux d'air ou la température au niveau des narines, soit de dispositifs dédiés enregistrant les déformations du thorax au moyen d'un transducteur associé à un capteur mécanique, hydraulique ou pneumatique et délivrant un signal électrique fonction desdites déformations.

Toutefois, en environnement IRM, ces dispositifs connus ne permettent pas de relever de manière précise, fiable et reproductible le rythme respiratoire du fait notamment de la nature même des grandeurs physiques mesurées, des perturbations électromagnétiques et des modes d'acquisition et de mesure utilisés, et de l'importance primordiale du positionnement des capteurs, souvent difficiles à installer correctement et fréquemment déplacés involontairement au cours de la séance de mesure par le patient.

Plus spécialement, la production de signaux électriques dans un environnement électromagnétique chargé et sensible, tel que celui d'un appareil à RMN, entraîne une perturbation néfaste dudit environnement et, réciproquement, une perturbation desdits signaux électriques de mesure produits, faussant leur signification.

De plus, les câbles ou autres conducteurs électriques, en formant antennes, perturbent l'environnement électromagnétique de l'appareil à RMN et faussent les mesures et, dans le cas d'un imageur, les reconstitutions virtuelles (images) obtenues par ce dernier, même lorsque lesdits câbles sont blindés et torsadés.

A l'inverse, les gradients de champ, les champs radiofréquence et les phénomènes liés aux commutations entre bobines émettrices et réceptrices au cours d'une expérience du type RMN, perturbent fortement la transmission des signaux relevés et peuvent, par la génération d'artefacts importants, rendre ces derniers totalement inexploitables, le patient étant disposé à l'intérieur même de l'aimant principal de l'appareil à RMN.

De plus, les mouvements éventuels du patient (notamment la respiration elle-même) entraînent des mouvements desdits câbles de transmission électrique dans le champ résident, d'où résulte automatiquement une induction de potentiels générateurs d'artefacts.

En outre, les phénomènes nuisibles précités sont fortement amplifiés lorsque les câbles de transmission présentent une ou plusieurs boucles.

Par ailleurs, la solution consistant à acquérir et à transmettre les informations ou signaux sous forme pneumatique ou hydraulique hors de l'environnement électromagnétique sensible n'est pas non plus satisfaisante du fait du manque de fiabilité et de précision de ces modes de transmission et des déperditions importantes qu'ils entraînent, des problèmes de positionnement du capteur et de la gêne occasionnée au patient.

De plus, les capteurs pneumatiques ou mécaniques sont tous sujet à de fortes dérives qu'il est nécessaire de compenser continuellement.

En outre, ces capteurs pneumatiques ou mécaniques ne réagissent qu'à un type donné de respiration, à savoir, thoracique ou diaphragmatique, et sont quasi-insensibles vis à vis de l'autre type, ce en fonction du positionnement desdits capteurs sur le patient.

Or, chez certains patients l'un seulement des deux types de respiration précités est largement prédominant, l'autre type étant d'amplitude insuffisante, voire négligeable.

Le problème posé à la présente invention consiste par conséquent à concevoir un dispositif capteur peu encombrant, relevant de manière précise et fiable le signal respiratoire ou correspondant aux mouvements respiratoires d'un patient et le mettant sous une forme transmissible sans perturbation dans un environnement électromagnétiquement chargé, ledit dispositif capteur devant pouvoir être mis en oeuvre en toute sécurité et sans influence réciproque sur un patient placé dans un appareil à RMN.

De plus, ledit dispositif capteur devra également, selon un second but de l'invention, pouvoir relever et transmettre indépendamment d'autres signaux physiologiques ou de commande d'autres appareils, sans augmenter notablement son encombrement ou la complexité de sa constitution.

Par ailleurs, le dispositif capteur à concevoir ne doit pas présenter de dérive, ou alors une dérive de faible ampleur et contrôlable, et pouvoir relever aussi bien les signaux générés par la respiration diaphragmatique, que ceux générés par la respiration thoracique.

A cet effet, la présente invention a pour objet un dispositif capteur destiné à être mis en oeuvre dans un environnement électromagnétique chargé et sensible, notamment à proximité ou à l'intérieur d'un appareil à résonance magnétique nucléaire, ledit dispositif capteur délivrant un signal représentatif de la respiration d'un patient, et plus particulièrement d'un patient situé à l'intérieur du tunnel de l'aimant d'un IRM, ledit dispositif capteur comprenant :
- un corps support en matériau amagnétique, comprenant une embase sur laquelle sont montées au moins deux électrodes non métalliques destinées à être appliquées sur la peau du patient dans la région cardiaque;
- une unité de traitement des signaux électriques émis par le coeur, comportant un premier module, d'acquisition et de mise en forme des signaux électriques cardiaques, un deuxième module, d'extraction du signal respiratoire à partir des signaux électriques cardiaques délivrés par ledit premier module, et un troisième module, de conversion électrooptique du signal respiratoire;
- un boîtier blindé formant cage de Faraday, porté par l'embase et renfermant ladite unité de traitement;
- un moyen de liaison optique pour relier ledit boîtier à au moins un autre appareil ou dispositif situé, le cas échéant, à l'extérieur de l'environnement électromagnétique chargé et sensible.

Selon un des modes de réalisation de l'invention, la structure d'ensemble du dispositif capteur est similaire à celle du dispositif capteur divulgué dans la publication WO94/23648. Ce dernier dispositif ne délivre cependant qu'un signal cardiaque, alors que le dispositif selon l'invention permet de délivrer directement un signal représentatif de la respiration du patient à partir du signal cardiaque, et ce au moyen de l'unité de traitement selon l'invention.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :
la figure 1 est une vue schématique externe, en élévation latérale, du dispositif capteur selon l'invention ;
la figure 2 est un schéma fonctionnel du dispositif capteur représenté à la figure 1 ;
la figure 3 est une représentation schématique montrant la mise en oeuvre du dispositif capteur représenté à la figure 1 ;
la figure 4 représente un signal d'électrocardiogramme disponible, après filtrage adapté, à la sortie de l'étage d'acquisition du premier module de l'unité de traitement faisant partie du dispositif capteur représenté à la figure 2 ;
la figure 5 représente, de manière schématique, simultanément les courbes du signal respiratoire R, du signal d'électrocardiogramme E et du signal de synchronisation S délivré par le circuit de détection cyclique selon l'invention, et,
la figure 6 est une vue en élévation latérale et en coupe d'un second mode de réalisation du capteur représenté à la figure 1.

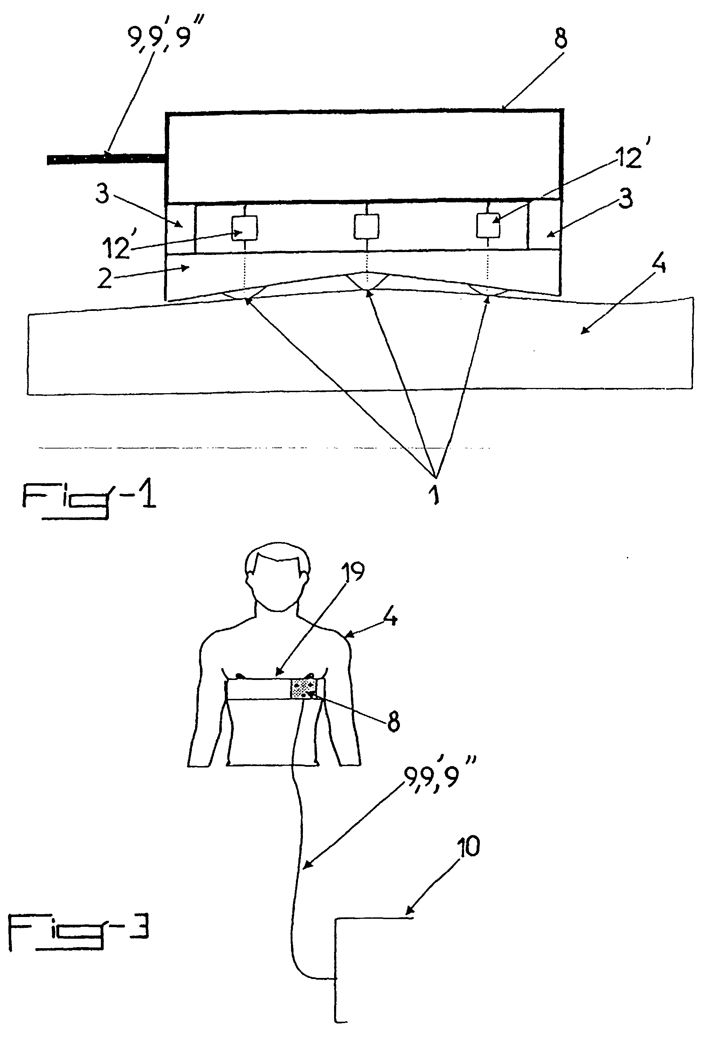

Conformément à l'invention, et comme le montrent les figures 1, 2 et 3 des dessins annexés, le dispositif capteur est principalement constitué, d'une part, par au moins deux électrodes 1 non métalliques, montées sur une embase 2 d'un corps support 3 en un matériau amagnétique et destinées à être appliquées sur la peau du patient 4 dans la région cardiaque, d'autre part, par une unité de traitement des signaux électriques émis par le coeur comportant un premier module 5 d'acquisition et de mise en forme des signaux électriques cardiaques, un deuxième module 6 d'extraction du signal respiratoire et un troisième module 7 de conversion électrooptique de ce dernier, disposés dans un boîtier blindé 8 formant cage de Faraday et porté par l'embase 2 et, enfin, par un moyen de liaison optique 9 reliant ledit boîtier 8 à au moins un autre appareil ou dispositif 10 situé, le cas échéant, à l'extérieur de l'environnement électromagnétique chargé et sensible.

La présente invention est par conséquent basée sur l'extraction du signal respiratoire à partir des signaux électrocardiographiques relevés de manière très précise par les électrodes 1 et immédiatement traités après leur prélèvement, sans transmission, dans une unité adaptée totalement isolée de l'environnement électromagnétique extérieur.

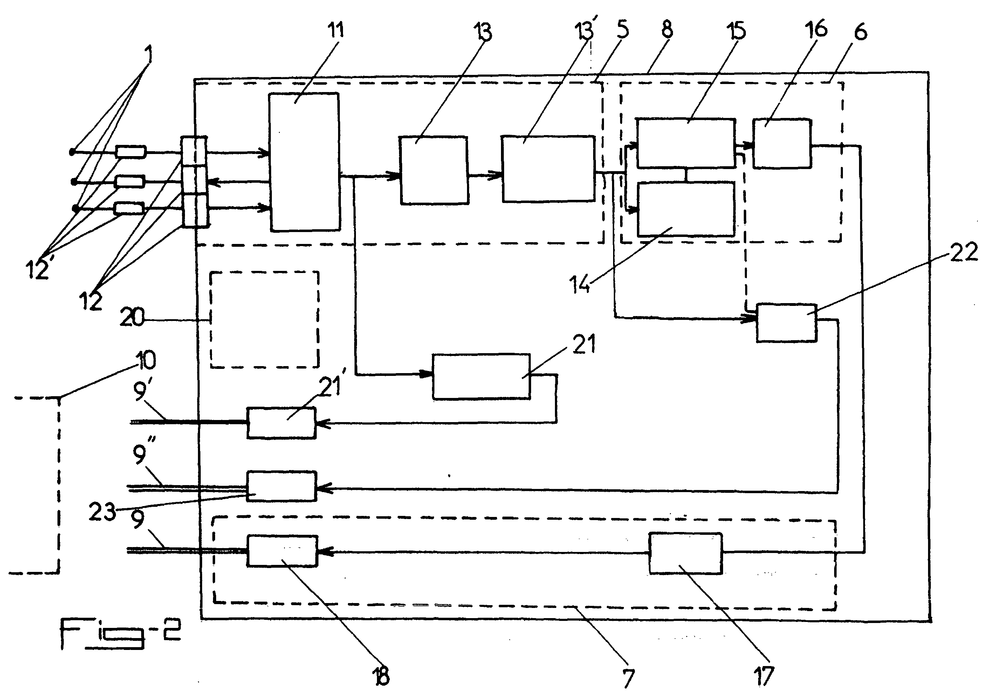

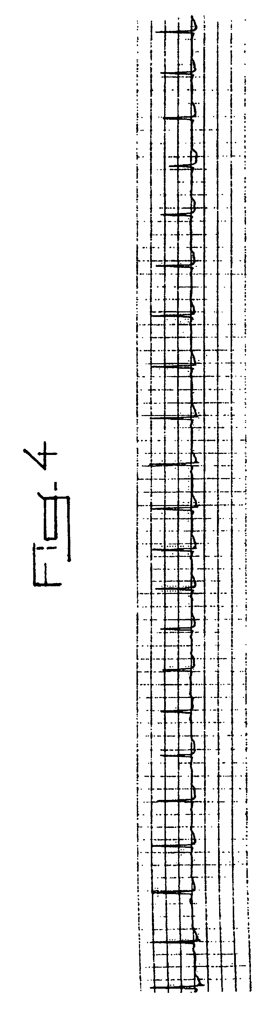

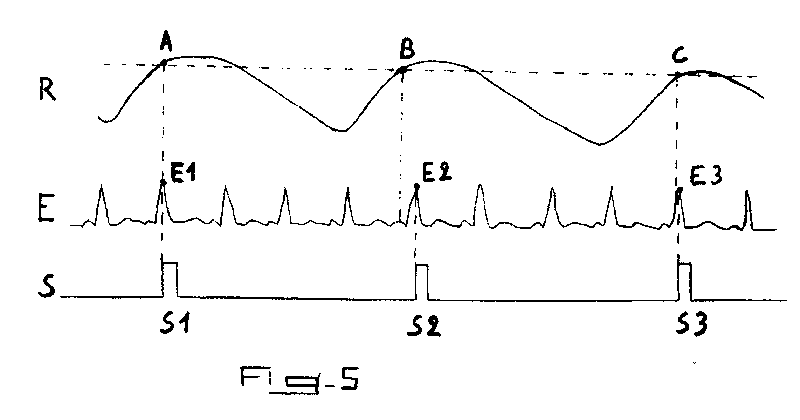

En effet, comme le montre notamment la figure 4 des dessins annexés, le signal d'électrocardiogramme, construit à partir des signaux électriques émis par le coeur et composé par une succession de complexes QRS, subit une variation d'amplitude périodique dont la phase et la période correspondent au cycle respiratoire.

Ce phénomène s'explique par le fait que, au cours du cycle respiratoire du patient, le coeur est déplacé périodiquement par les mouvements de dilatation et de contraction successifs de la cage thoracique et les mouvements résultant des autres organes qui y sont logés, ce qui entraîne directement un déplacement de l'axe électrique du coeur.

C'est donc cette répercution directe des mouvements respiratoires cycliques sur le signal d'électrocardiogramme qui est mise à profit par l'invention pour extraire de ce dernier le signal respiratoire.

Selon une première caractéristique de l'invention, le premier module 5 comprend, d'une part, un étage d'acquisition des signaux électrocardiaques relevés par les électrodes 1, constitué essentiellement par un amplificateur d'instrumentation 11 dont chacune des entrées est reliée à une électrode 1 correspondante par l'intermédiaire d'un filtre radiofréquence 12 et d'une résistance de limitation 12', et, d'autre part, un étage de mise en forme composé d'un filtre passe-bande 13 et d'un circuit redresseur 13'.

Les filtres 12 et les résistances 12', disposés entre l'amplificateur d'instrumentation 11 et les électrodes 1, ont pour rôle, respectivement, d'éliminer les parasites induits par les émissions radiofréquences environnantes et de réduire les courants éventuellement induits par ces dernières, les liaisons entre les électrodes 1 et le boîtier 8 étant en outre réalisées au moyen de fils rigides.

De manière avantageuse, lesdites électrodes 1 sont au nombre de trois et sont réalisées en un matériau conducteur choisi dans le groupe formé par le carbone, les composés de carbone et les matières plastiques chargées, l'une desdites électrodes 1 étant utilisée pour augmenter le taux de réjection du mode commun.

En outre, les électrodes 1 étant fixées sur l'embase 2, les emplacements relatifs des unes par rapport aux autres sont figés, en étant écartés d'une distance qui peut être fonction de la taille du patient et il suffit de positionner ladite embase 2 à proximité du coeur pour que les électrodes 1 soient placées de manière satisfaisante.

L'unique élément métallique externe du dispositif capteur, à savoir le boîtier blindé 8, ne sera, par conséquent, jamais en contact direct avec le patient 4, puisque au moins séparé de ce dernier par ladite embase 2, ce qui évite tout risque de brûlure.

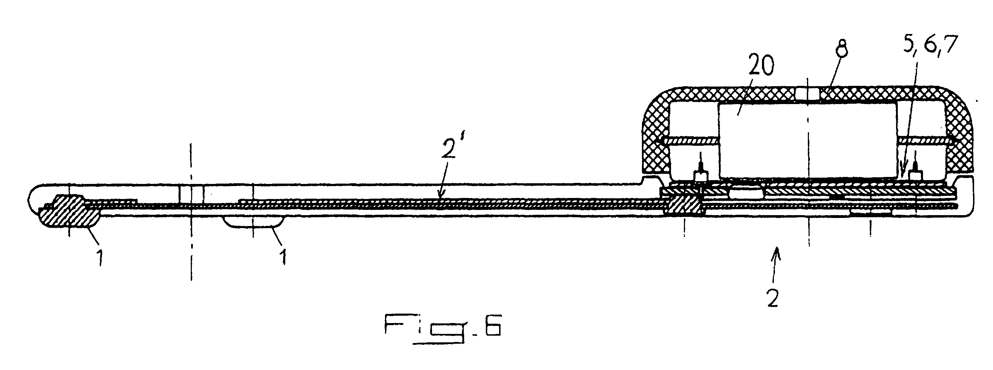

Conformément à un second mode de réalisation du dispositif capteur selon l'invention, représenté à la figure 6 des dessins annexés, les électrodes 1 ne sont pas positionnées sous le boîtier 8, mais déportées par rapport à ce dernier et fixées sur l'extrémité d'un prolongement latéral 2', de faible épaisseur, de l'embase 2, la liaison entre lesdites électrodes 1 et le boîtier 8 étant réalisée par des conducteurs non métalliques, par exemple en fibres de carbone.

Une telle construction du dispositif capteur permet d'éloigner le boîtier 8, qui a une hauteur ou épaisseur non négligeable, des points de recueil de l'ECG dans la région cardiaque, où il peut être nécessaire, le cas échéant, d'appliquer une antenne de mesure IRM au plus près de la peau du patient.

De manière avantageuse, le filtre passe-bande 13 du premier module 5 permet de débarrasser le signal délivré par l'amplificateur d'instrumentation 11 de ses composantes superflues et inutiles pour le traitement ultérieur et d'isoler les parties du signal (les complexes QRS successifs) dont on veut mesurer la variation d'amplitude avec la respiration.

Ce filtre passe-bande 13 peut par exemple être constitué par l'association d'un filtre passe-bas et d'un filtre passe-haut, délimitant ensemble une bande passante de 15 Hz à 35 Hz environ.

Par ailleurs, le circuit redresseur 13' permet d'obtenir à la sortie dudit module 5 un signal toujours polarisé dans un sens connu et déterminé. En effet, en fonction du positionnement des électrodes 1 par rapport au corps, le signal d'électrocardiogramme délivré par l'amplificateur d'instrumentation 11 peut être de polarité différente et opposée.

Comme le montre la figure 2 des dessins annexés, le deuxième module 6 comprend préférentiellement un circuit 14 détecteur de maxima, alimenté par le signal mis en forme issu du premier module 5 et commandant un circuit échantillonneur-bloqueur 15 alimenté également par le signal issu dudit premier module 5, le signal de sortie dudit circuit échantillonneur-bloqueur 15 étant traité par un circuit moyenneur et lisseur 16.

Ainsi, le circuit détecteur 14 permet de détecter les moments d'apparition des signaux mis en forme ou conditionnés par le dernier étage du premier module 5, signaux dont on veut mesurer l'amplitude d'un point déterminé, par exemple l'amplitude maximale.

Cette détection est utilisée pour commander le circuit échantillonneur-bloqueur 15 qui réalise la mesure et assure le maintien des amplitudes relevées qui varient avec les complexes QRS successifs en fonction des cycles respiratoires. Le signal issu dudit circuit 15 se présente sous la forme d'une succession d'échelons d'amplitudes variables avec la respiration et représentant schématiquement le signal respiratoire.

Cette structure permet d'éviter que le signal issu du circuit 15 ne soit affecté par les artefacts de tension pouvant exister au niveau du signal issu de l'amplificateur d'instrumentation 11 entre deux mesures successives d'amplitudes et qui n'ont aucun rapport avec l'activité cardiaque et dont l'amplitude n'est en aucun cas liée à l'activité respiratoire.

Le circuit 16 calcule la valeur moyenne du signal issu de l'échantillonneur-bloqueur 15 et en supprime la composante de tension continue, le signal délivré par ce circuit représentant par conséquent de manière précise le signal respiratoire.

En variante, le deuxième module 6 peut se présenter sous la forme d'un circuit intégré digital ou de plusieurs circuits intégrés digitaux programmables réalisant un traitement numérique des signaux délivrés par le premier module 5 et fournissant un signal représentatif de la respiration ou un signal de synchronisation dérivé du signal respiratoire au troisième module 7.

Conformément à une autre caractéristique de l'invention, représentée également à la figure 2 des dessins annexés, le troisième module 7 est avantageusement composé d'un circuit modulateur 17, par exemple à modulation de fréquence ou de largeur d'impulsion, recevant le signal délivré par le deuxième module 6 et d'un transducteur électrooptique 18 relié à un conducteur optique 9 constituant le moyen de liaison optique.

Ce dernier pourra délivrer le signal respiratoire, par exemple, à un dispositif de visualisation et d'affichage, à un moyen de commande de l'appareil à RMN ou d'un dispositif annexe, ou à plusieurs de ces appareils, dispositifs ou analogues après dérivation ou multiplexage dudit conducteur optique.

Conformément à un mode de réalisation préféré de l'invention représenté à la figure 3 des dessins annexés et afin d'assurer un positionnement ferme des électrodes 1 et de garantir un prélèvement des signaux électriques cardiaques aussi près que possible du coeur, il est prévu une ceinture 19 ou un harnais en un matériau amagnétique, éventuellement élastique, pourvu d'un moyen de fermeture rapide et de réglage de la longueur et traversant le corps support 3 ou au moins une anse solidaire dudit corps support 3, ladite ceinture 19 ou harnais assurant le positionnement en translation et en rotation dudit corps support 3 et desdites électrodes 1.

Ainsi, l'embase 2 du corps support 3 et donc les électrodes 1 seront, en permanence, appliqués à force contre la peau du patient au niveau de la région du coeur.

Selon une première variante de réalisation de l'invention, le boîtier blindé 8 contient, en outre, une batterie 20 ou un accumulateur rechargeable longue durée et de type amagnétique, un conducteur optique, associé à un interrupteur à commande optique disposé dans le boîtier 8, pouvant permettre de contrôler le fonctionnement et l'alimentation de ladite unité de traitement 5, 6, 7 et, le cas échéant, le réglage des différents circuits composant ses modules constitutifs (non représenté).

Selon une seconde variante de réalisation de l'invention, l'alimentation en énergie de l'unité de traitement est réalisée au moyen d'un conducteur optique coopérant avec une cellule photovoltaïque ou un dispositif similaire disposé dans le boîtier.

Conformément à une caractéristique supplémentaire de l'invention, représentée à la figure 2 des dessins annexés, le dispositif capteur peut également comporter une unité indépendante supplémentaire de traitement des signaux délivrés par l'étage d'acquisition du premier module 5, composée essentiellement d'un filtre passe-bas 21, présentant une fréquence de coupure à environ 20 Hz, et d'un module 21' de conversion électrooptique relié à un second moyen de liaison optique 9'.

Une telle unité de traitement supplémentaire est notamment décrite dans la demande de brevet français n° 2 704 131 au nom de la demanderesse.

Ainsi, grâce à cette dernière disposition, il sera possible de délivrer indépendamment deux signaux physiologiques importants de natures différentes, élaborés à partir des mêmes signaux électrocardiaques de départ, par des traitements séparés et notamment un filtrage adapté à chacune des voies de traitement, réalisés à proximité immédiate de l'endroit de prélèvement des signaux de départ.

Selon une autre caractéristique supplémentaire de l'invention, le dispositif capteur peut comporter, en outre, un circuit 22 de détection cyclique d'un point ou d'un niveau déterminé du signal répétitif (électrocardiogramme modulé en amplitude par la respiration) délivré par le premier module 5 et de fourniture d'un signal de synchronisation à chaque occurrence et détection dudit point ou niveau déterminé, ledit circuit de détection 22 étant suivi d'un convertisseur électrooptique 23 correspondant relié à un moyen de liaison optique propre 9".

En variante, ledit circuit de détection 22 pourra également être alimenté par le signal issu du circuit échantillonneur-bloqueur 15 faisant partie du second module 6.

Les signaux de synchronisation délivrés par le circuit de détection 22 correspondront aux coïncidences approximatives successives, répétées périodiquement, entre un point donné d'un complexe QRS (par exemple son maximum) du signal d'électrocardiogramme et un point donné du signal respiratoire.

Ce signal, qui constitue un repère temporel, permettra par conséquent de déterminer temporellement et de repérer de manière répétitive un état donné du coeur dans une position donnée de ce dernier, au cours du cycle respiratoire.

Le mode de fonctionnement du circuit 22 et la signification du signal fourni par ce dernier peuvent être expliqués plus précisément en rapport avec la figure 5 des dessins annexés.

En prenant comme référence le signal respiratoire, on peut y définir un emplacement temporel A coïncidant avec un complexe QRS E1 et rechercher dans la période suivante du signal respiratoire, au même endroit du cycle B, le complexe QRS E2 le plus proche, et ainsi de suite pour les cycles suivants (cycle C et QRS E3, etc.).

A partir des QRS E1, E2, E3, etc., on pourra dériver des impulsions de synchronisation de l'image (également connues sous l'appellation "signaux TRIGGER") tels que S1, S2, S3, etc., tenant directement compte des deux activités cardiaque et respiratoire.

Les images RMN pourront également être réalisées avec des décalages de temps constants par rapport aux références de temps correspondant aux impulsions S1, S2, S3, etc.

Un tel signal de synchronisation, indiquant les occurrences successives d'un point ou d'un niveau déterminé dans le signal respiratoire, est donc particulièrement intéressant dans le cadre de l'imagerie RMN pour déclencher les séquences répétitives de prises d'images, formant par superpositions et reconstitution virtuelle l'image finale.

En variante à l'exploitation des signaux d'ECG et de mouvements respiratoires telle que décrite ci-dessus dans le cadre de réalisation d'image RNM, il peut être prévu de réaliser des images RMN à chaque occurrence d'un complexe QRS et en utilisant chacun de ces derniers en tant qu'impulsions de synchronisation de l'image, le signal respiratoire étant prélevé et enregistré simultanément et séparément par le dispositif capteur.

Ensuite, lors de l'exploitation ultérieure des images RMN réalisées, le signal respiratoire est utilisé pour extraire et sélectionner les images RMN qui correspondent à un endroit déterminé et répétitif du cycle respiratoire (compensation respiratoire).

Ainsi, le dispositif capteur selon l'invention permet de synchroniser la prise d'images RMN et/ou la formation d'images RMN sur la base de deux signaux physiologiques, à savoir le signal d'ECG et le signal respiratoire, et donc de tenir compte des deux types de mouvements physiologiques pour la formation desdites images.

## Revendications

1. Dispositif capteur destiné à être mis en oeuvre dans un environnement électromagnétique chargé et sensible, notamment à proximité ou à l'intérieur d'un appareil à résonance magnétique nucléaire, ledit dispositif capteur délivrant un signal représentatif de la respiration d'un patient, et plus particulièrement d'un patient situé à l'intérieur du tunnel de l'aimant d'un IRM, ledit dispositif capteur comprenant :
- un corps support (3) en matériau amagnétique, comprenant une embase (2) sur laquelle sont montées au moins deux électrodes (1) non métalliques destinées à être appliquées sur la peau du patient (4) dans la région cardiaque;
- une unité de traitement des signaux électriques émis par le coeur, comportant un premier module (5), d'acquisition et de mise en forme des signaux électriques cardiaques, un deuxième module (6), d'extraction du signal respiratoire à partir des signaux électriques cardiaques délivrés par ledit premier module, et un troisième module (7), de conversion électrooptique du signal respiratoire;
- un boîtier blindé (8) formant cage de Faraday, porté par l'embase et renfermant ladite unité de traitement;
- un moyen de liaison optique (9) pour relier ledit boîtier à au moins un autre appareil ou dispositif (10) situé, le cas échéant, à l'extérieur de l'environnement électromagnétique chargé et sensible.

2. Dispositif capteur selon la revendication 1, **caractérisé en ce que** le premier module (5) comprend, d'une part, un étage d'acquisition des signaux électrocardiaques relevés par les électrodes (1), constitué essentiellement par un amplificateur d'instnunentation (11) dont chacune des entrées est reliée à une électrode (1) correspondante par l'intermédiaire d'un filtre radiofréquence (12) et d'une résistance de limitation (12'), et, d'autre part, un étage de mise en forme composé d'un filtre passe-bande (13) et d'un circuit redresseur (13').

3. Dispositif capteur selon l'une quelconque des revendications 1 et 2, **caractérisé en ce que** le deuxième module (6) comprend un circuit (14) détecteur de maxima, alimenté par le signal mis en forme issu du premier module (5), un circuit échantillonneur-bloqueur (15) alimenté également par le signal issu dudit premier module (5) et commandé par le circuit détecteur de maxima, et un circuit moyenneur et lisseur (16) traitant le signal de sortie dudit circuit échantillonneur.

4. Dispositif capteur selon l'une quelconque des revendications 1 à 3, **caractérisé en ce que** le troisième module (7) est composé d'un circuit modulateur (17), par exemple à modulation de fréquence ou de largeur d'impulsion, recevant le signal délivré par le deuxième module (6) et d'un transducteur électrooptique (18) relié à un conducteur optique (9) constituant le moyen de liaison optique.

5. Dispositif capteur selon l'une quelconque des revendications 1 à 4, **caractérisé en ce que** les électrodes (1) sont au nombre de trois et sont réalisées en un matériau conducteur choisi dans le groupe formé par le carbone, les composés de carbone et les matières plastiques chargées, l'une desdites électrodes (1) étant utilisée pour augmenter le taux de réjection en mode commun.

6. Dispositif capteur selon l'une quelconque des revendications 1 à 5, **caractérisé en ce qu'**il comprend une ceinture (19) ou un harnais en un matériau amagnétique, éventuellement élastique, pourvu d'un moyen de fermeture rapide et de réglage de la longueur et traversant le corps support (3) ou au moins une anse solidaire dudit corps support (3), ladite ceinture (19) ou harnais assurant le positionnement en translation et en rotation dudit corps support (3) et desdites électrodes (1).

7. Dispositif capteur selon l'une quelconque des revendications 1 à 6, **caractérisé en ce que** le boîtier blindé (8) contient, en outre, une batterie (20) ou un accumulateur rechargeable longue durée et de type amagnétique, un conducteur optique, associé à un interrupteur à commande optique disposé dans le boîtier (8), pouvant permettre de contrôler le fonctionnement et l'alimentation de ladite unité de traitement (5, 6, 7) et, le cas échéant, le réglage des différents circuits composant ses modules constitutifs.

8. Dispositif capteur selon l'une quelconque des revendications 1 6, **caractérisé en ce que** l'alimentation en énergie de l'unité de traitement est réalisée au moyen d'un conducteur optique coopérant avec une cellule photovoltaïque ou un dispositif similaire disposé dans le boîtier.

9. Dispositif capteur selon l'une quelconque des revendications 2 à 8, **caractérisé en ce qu'**il comporte une unité indépendante supplémentaire de traitement des signaux délivrés par l'étage d'acquisition du premier module (5), composée essentiellement d'un filtre passe-bas (21), présentant une fréquence de coupure à environ 20 Hz, et d'un module (21') de conversion électrooptique relié à un second moyen de liaison optique (9')

10. Dispositif capteur selon l'une quelconque des revendications 3 à 9, **caractérisé en ce qu'**il comporte, en outre, un circuit (22) de détection cyclique d'un point ou d'un niveau déterminé du signal répétitif délivré par l'étage d'acquisition du premier module (5) et de fourniture d'un signal de synchronisation à chaque occurrence et détection dudit point ou niveau déterminé, ledit circuit de détection (22) étant suivi d'un convertisseur électrooptique (23) correspondant relié à un moyen de liaison optique propre (9").

11. Dispositif capteur selon la revendication 10, **caractérisé en ce que** le circuit de détection cyclique (22) est alimenté par le signal issu du circuit échantillonneur-bloqueur (15) faisant partie du second module (6).

12. Dispositif capteur selon l'une quelconque des revendications 1 à 11, **caractérisé en ce que** les électrodes (1) sont déportées par rapport au boîtier blindé (8) et sont fixées sur l'extrémité d'un prolongement latéral (2'), de faible épaisseur, de l'embase (2), la liaison entre lesdites électrodes (1) et le boîtier (8) étant réalisée par des conducteurs non métalliques, par exemple en fibres de carbone.

13. Utilisation d'un dispositif capteur selon l'une quelconque des revendications 11 et 12, **caractérisée en ce que** le signal de synchronisation indiquant les occurrences successives d'un point ou d'un niveau déterminé dans le signal respiratoire est utilisé pour déclencher les séquences répétitives de prises d'images RMN, formant par superposition et reconstitution virtuelle l'image finale.

## Patentansprüche

1. Sensorvorrichtung zum Betrieb in einer mit einem elektromagnetischen Feld durchsetzten und auf dieses empfindlichen Umgebung, insbesondere in der Nähe von oder innerhalb einer Kernspinresonanzvorrichtung, wobei die Sensorvorrichtung ein für die Atmung eines Patienten, insbesondere eines im Innern des Tunnels des Magneten bei der Kernspintomografie befindlichen Patienten, signifikantes Signal liefert, wobei die Sensorvorrichtung
- einen Trägerkörper (3) aus einem nichtmagnetischen Material mit einem Sockel (2), an dem wenigstens zwei nichtmetallische Elektroden (1) angebracht sind, die dazu bestimmt sind, auf der Haut des Patienten (4) in dem kardiologisch relevanten Bereich appliziert zu werden,
- eine Einheit zum Bearbeiten von durch das Herz abgegebenen elektrischen Signalen mit einem ersten Modul (5) zum Aufnehmen und Aufbereiten der kardiologischen elektrischen Signale, mit einem zweiten Modul (6) zur Extraktion des Atmungssignals aus den durch das erste Modul bereitgestellten kardiologischen elektrischen Signale und mit einem dritten Modul (7) zum elektrooptischen Umwandeln des Atmungssignals,
- ein abgeschirmtes, aus einem Faraday'schen Käfig gebildetes Gehäuse (8), das durch den Sockel getragen ist und die Bearbeitungseinheit umschließt, und
- ein optisches Verbindungsmittel (9) zum Verbinden des Gehäuses mit wenigstens einem weiteren Gerät oder einer Einheit (10), das beziehungsweise die gegebenenfalls außerhalb der elektromagnetisch durchsetzten und empfindlichen Umgebung angeordnet ist,
aufweist.

2. Sensorvorrichtung nach Anspruch 1, **dadurch gekennzeichnet, dass** das erste Modul (5) einerseits eine Stufe zum Sammeln der durch die Elektroden (1) aufgenommenen elektrokardiologischen Signale aufweist, die im wesentlichen durch einen Instrumentationsverstärker (11) gebildet ist, bei dem jeder der Eingänge über einen Radiofrequenzfilter (12) und einen Begrenzungswiderstand (12') an eine zugehörige Elektrode (1) angeschlossen ist, und weiterhin eine aus einem Tiefpassfilter (13) und einem Gleichrichtschaltkreis (13') gebildete Aufbereitungsstufe aufweist.

3. Sensorvorrichtung nach einem der Ansprüche 1 und 2, **dadurch gekennzeichnet, dass** das zweite Modul (6) einen Maximumsdetektionsschaltkreis (14), der durch das aufbereitete, durch das erste Modul (5) abgegebene Signal gespeist ist, einen ebenfalls durch das von dem ersten Modul (5) abgegebene Signal gespeisten sowie durch den Maximumdetektionsschaltkreis gesteuerten Sample- und Hold-Schaltkreis und einen mittelnden und glättenden Schaltkreis (16) aufweist, der das Ausgangssignal des Sample- und Hold-Schaltkreises verarbeitet.

4. Sensorvorrichtung nach einem der Ansprüche 1 bis 3, **dadurch gekennzeichnet, dass** das dritte Modul (7) aus einem Modulationsschaltkreis (17), beispielsweise zur Modulation der Frequenz oder der Pulshöhe, der das durch das zweite Modul (6) gelieferte Signal aufnimmt, und aus einem elektrooptischen Signalwandler (18) gebildet ist, der mit einem das optische Verbindungsmittel bildenden optischen Leiter (9) verbunden ist.

5. Sensorvorrichtung nach einem der Ansprüche 1 bis 4, **dadurch gekennzeichnet, dass** die Elektroden (1) dreifach vorhanden und aus einem leitenden Material aus der durch Kohlenstoff, Kohlenstoffverbindungen und elektrisch leitenden Kunststoffmaterialien gebildeten Gruppe ausgeführt sind, wobei eine der Elektroden (1) dazu verwendet wird, die Gleichtaktunterdrückung zu verbessern.

6. Sensorvorrichtung nach einem der Ansprüche 1 bis 5, **dadurch gekennzeichnet, dass** sie einen Gürtel (19) oder ein Gurtzeug aus einem nichtmagnetischen, unter Umständen auch elastischen Material aufweist, der beziehungsweise das mit einem Schnellverschluss- und Längenverstellmittel ausgestattet ist und durch den Trägerkörper (3) oder wenigstens durch einen mit dem Trägerkörper (3) fest verbundenen Befestigungsbügel verläuft, wobei der Gürtel (19) oder das Gurtzeug das Anordnen des Trägerkörpers (3) und der Elektroden (1) in Translation und in Rotation sicherstellt.

7. Sensorvorrichtung nach einem der Ansprüche 1 bis 6, **dadurch gekennzeichnet, dass** das abgeschirmte Gehäuse (8) weiterhin eine Batterie (20) oder einen wieder aufladbaren Akkumulator mit langer Lebensdauer sowie des nichtmagnetischen Typs und einen optischen Leiter enthält, der mit einem innerhalb des Gehäuses (8) angeordneten, optisch ansteuerbaren Schalter verbunden ist, mit dem der Betrieb und die Versorgung der Bearbeitungseinheit (5, 6, 7) und gegebenenfalls die Steuerung der die verschiedenen Module bildenden Schaltkreise kontrollierbar ist.

8. Sensorvorrichtung nach einem der Ansprüche 1 bis 6, **dadurch gekennzeichnet, dass** die Energieversorgung der Bearbeitungseinheit mittels eines optischen Leiters ausgeführt ist, der mit einer photovoltaischen Zelle oder einer ähnlichen, innerhalb des Gehäuses angeordneten Vorrichtung zusammenwirkt.

9. Sensorvorrichtung nach einem der Ansprüche 2 bis 8, **dadurch gekennzeichnet, dass** sie eine zusätzliche unabhängige Bearbeitungseinheit für die von der Aufnahmestufe des ersten Moduls (5) gelieferten Signale aufweist, die im wesentlichen aus einem Tiefpassfilter (21) mit einer Grenzfrequenz von etwa 20 Hz und aus einem mit dem zweiten optischen Verbindungsmittel (9') verbundenen elektrooptischen Umwandlungsmodul (21') gebildet ist.

10. Sensorvorrichtung nach einem der Ansprüche 3 bis 9, **dadurch gekennzeichnet, dass** sie weiterhin einen Schaltkreis (22) zum zyklischen Detektieren eines vorbestimmten Punktes oder Pegels des repetitiv durch die Aufnahmestufe des ersten Moduls (5) gelieferten Signals und zum Bereitstellen eines Synchronisationssignales bei jedem Auftreten und Detektieren des vorbestimmten Punktes oder Pegels aufweist, wobei dem Detektionsschaltkreis (22) ein mit einem zugehörigen eigenen optischen Verbindungsmittel (9") verbundener elektrooptischer Wandler (23) nachgeordnet ist.

11. Sensorvorrichtung nach Anspruch 10, **dadurch gekennzeichnet, dass** der zyklisch arbeitende Detektionsschaltkreis (22) durch das von dem einen Teil des zweiten Moduls (6) bildenden Sample- und Hold-Schaltkreis (15) gelieferte Signal gespeist wird.

12. Sensorvorrichtung nach einem der Ansprüche 1 bis 11, **dadurch gekennzeichnet, dass** die Elektroden (1) in Bezug auf das abgeschirmte Gehäuse (8) versetzt angeordnet und an dem Ende einer seitlichen Verlängerung (2') mit geringer Dicke angeordnet sind, wobei die Verbindung zwischen den Elektroden (1) und dem Gehäuse (8) durch nichtmetallische Leiter, beispielsweise Kohlenstofffasern, ausgeführt ist.

13. Verwendung einer Sensorvorrichtung nach einem der Ansprüche 11 und 12, **dadurch gekennzeichnet, dass** das das wiederholte aufeinanderfolgende Auftreten eines vorbestimmten Punktes oder Pegels in dem Atmungssignal anzeigende Synchronisationssignal verwendet wird, um die sich wiederholenden, durch Überlagerung und virtuelle Wiederherstellung das endgültige Bild ergebenden Kernspinresonanzbildaufnahmesequenzen auszulösen.

## Claims

1. A sensor intended for use in a charged and sensitive electromagnetic environment, especially in proximity to or within a nuclear magnetic resonance apparatus, said sensor delivering a signal representative of the respiration of a patient, and more particularly of a patient located within the tunnel of the magnet of an MRI, said sensor comprising:
- a support body (3) made of non-magnetic material, comprising a base (2) on which are mounted at least two non-metallic electrodes (1) intended to be applied to the skin of the patient (4) in the cardiac region;
- a processing unit for processing the electrical signals emitted by the heart, including a first module (5), for acquisition and transformation of the cardiac electrical signals, a second module (6), for extraction of the respiratory signal from the cardiac electrical signals delivered by said first module, and a third module (7), for electro-optical conversion of the respiratory signal;
- a shielded housing (8) forming a Faraday cage, borne by the base and enclosing said processing unit;
- an optical connection means (9) for connecting said housing to at least one other apparatus or device (10) located, if required, outside the charged and sensitive electromagnetic environment.

2. A sensor according to claim 1, **characterised in that** the first module (5) comprises, on the one hand, an acquisition stage for acquiring the electrocardiac signals picked up by the electrodes (1), and constituted essentially by an instrumentation amplifier (11), each of the inputs of which is connected to a corresponding electrode (1) by way of a radiofrequency filter (12) and a limitation resistance (12') and, on the other hand, a transformation stage composed of a band-pass filter (13) and of a rectifier circuit (13').

3. A sensor according to claim 1 or claim 2, **characterised in that** the second module (6) comprises a maxima detection circuit (14), fed by the transformed signal issuing from the first module (5), a sampler/blocker circuit (15) also fed by the signal issuing from said first module (5) and controlled by the maxima detection circuit, and an averaging and smoothing circuit (16) processing the output signal of said sampler/blocker circuit.

4. A sensor according to any one of claims 1 to 3, **characterised in that** the third module (7) is composed of a modulator circuit (17), for example with frequency modulation or pulse width modulation, receiving the signal delivered by the second module (6), and of an electro-optical transducer (18) connected to an optical conductor (9) constituting the optical connection means.

5. A sensor according to any one of claims 1 to 4, **characterised in that** the electrodes (1) are three in number and are made of a conducting material selected from the group formed by carbon, carbon compounds and charged plastics materials, one of said electrodes (1) being used to increase the common mode rejection ratio.

6. A sensor according to any one of claims 1 to 5, **characterised in that** it comprises a belt (19) or a harness made of a non-magnetic material, elastic if required, provided with a means for quick closure and for adjustment of the length and passing through the support body (3) or at least a loop rigidly connected to the support body (3), said belt (19) or harness effecting the positioning in translation and in rotation of said support body (3) and of said electrodes (1).

7. A sensor according to any one of claims 1 to 6, **characterised in that** the shielded housing (8) further contains a battery (20) or a rechargeable battery with long duration and of non-magnetic type, an optical conductor, associated with an optically controlled switch disposed in the housing (8), capable of making it possible to control the functioning and the supply of said processing unit (5, 6, 7) and, if required, the adjustment of the different circuits forming its constituent modules.

8. A sensor according to any one of claims 1 to 6, **characterised in that** the supply of power to the processing unit is effected by means of an optical conductor co-operating with a photovoltaic cell or a similar device disposed in the housing.

9. A sensor according to any one of claims 2 to 8, **characterised in that** it includes a supplementary independent processing unit for processing the signals delivered by the acquisition stage of the first module (5), composed essentially of a low-pass filter (21), having a cut-off frequency at approximately 20 Hz, and of an electro-optical conversion module (21') connected to a second optical connection means (9').

10. A sensor according to any one of claims 3 to 9, **characterised in that** it further includes a cyclic detection circuit (22) for detecting a specific point or level of the repetitive signal delivered by the acquisition stage of the first module (5) and for supplying a synchronisation signal at each occurrence and detection of said specific point or level, said detection circuit (22) being followed by a corresponding electro-optical converter (23) connected to its own optical connection means (9").

11. A sensor according to claim 10, **characterised in that** the cyclic detection circuit (22) is fed by the signal issuing from the sampler/blocker circuit (15) forming part of the second module (6).

12. A sensor according to any one of claims 1 to 11, **characterised in that** the electrodes (1) are off-set with respect to the shielded housing (8) and are fixed on the end of a thin, lateral extension (2') of the base (2), the connection between said electrodes (1) and the housing (8) being effected by non-metallic conductors, for example made of carbon fibres.

13. The use of a sensor according to claim 11 or claim 12, **characterised in that** the synchronisation signal indicating the consecutive occurrences of a specific point or level in the respiratory signal is used to trigger the repetitive sequences of NMR image taking, forming the final image by superposition and virtual reconstitution.
